# EUROPEAN PATENT APPLICATION

(11) **EP 4 786 982 A1**
(43) Date of publication of application: **05.08.2026**
(21) Application number: 24872384.3
(22) Date of filing: 26.09.2024
(51) Int. Cl.: G01N 33/543, G01N 33/531

(54) **LATEX PARTICLE DISPERSION, REAGENT KIT, DETECTION METHOD, PRECIPITATION SUPPRESSING AGENT, AND METHOD FOR SUPPRESSING PRECIPITATION**

(30) Priority: 27.09.2023 JP 2023165930
(71) Applicant: Sekisui Medical Co., Ltd., Tokyo 103-0027 (JP)
(72) Inventor: KAGAYA Hiroshi, Tokyo 103-0027 (JP); MATSUMOTO Suguru, Tokyo 103-0027 (JP)
(74) Representative: Ter Meer Steinmeister & Partner
(86) International application number: PCT/JP2024/034405
(87) International publication number: WO 2025/070599

(57) **Abstract**

The present invention relates to a latex particle dispersion containing latex particles and an aqueous medium, and also containing at least one compound selected from the group consisting of hexametaphosphoric acid and salts thereof.

## Description

### Technical Field

The present invention relates to a latex particle dispersion, a reagent kit, a detection method, a sedimentation suppressing agent, and a method for suppressing sedimentation.

Priority is claimed on Japanese Patent Application No. 2023-165930, filed September 27, 2023, the content of which is incorporated herein by reference.

### Background Art

Reagents or reagent kits (hereinafter also referred to as "detection reagents and the like") for detecting a target substance using a latex turbidimetric immunoassay method (LTIA method, also known as a "latex immunoagglutination assay method") can be mounted in general-purpose automatic analyzers, and exhibit excellent rapidity, accuracy, and profitability. Moreover, as a result of improvements in detection sensitivity, these detection reagents and the like can now offer dramatically improved contributions to clinical testing.

Detection reagents and the like are generally composed of two types of reagents, namely a first reagent (a buffer solution) and a second reagent (a latex particle dispersion). The first reagent has the roles of uniformly diluting the specimen (hereinafter also referred to as a "sample") that represents the analysis target, enabling the agglutination reaction caused by the latex particles to proceed uniformly without bias. The second reagent contains monoclonal antibody-sensitized latex particles as the main reaction component, which are a combination of monoclonal antibodies having a high specificity for the target substance such as an antigen, and latex particles capable of maintaining the characteristics of the sensitizing antibodies to a high degree. The second reagent also contains an aqueous medium for dispersing the latex particles, and a preservative and the like. The second reagent is also known as a latex reagent.

By using a detection reagent with an automatic analyzer, multiple samples can be measured simply and rapidly. The detection reagent is housed in a container and stored inside a reagent storage chamber in the automatic analyzer. At this time, from the viewpoint of usability, it is desirable that the reagent composition remains uniform for long periods when sitting in the container inside the reagent storage chamber, namely, exhibits favorable on-board stability.

However, in a detection reagent used in the LTIA method, if the particle size of the latex particles contained in the latex reagent is increased to improve the sensitivity, then once the latex reagent is placed in the container, the latex particles may sometimes sediment over time, causing a loss of uniformity in the latex reagent. If a sample is measured using this type of non-uniform latex reagent, an accurate measurement value may sometimes not be obtainable. Accordingly, in order to enable accurate measurements to be made using a reagent containing latex particles, redispersion of the latex particles in the aqueous medium by inverting the latex reagent container or stirring the latex reagent prior to measurement has typically been necessary.

Patent Document 1 discloses an object of "providing a method for suppressing the sedimentation of reactive substance-bound fine particles in a dispersion of those fine particles", and discloses that this object can be achieved by "a method for suppressing the sedimentation of fine particles to which a substance having reactivity is bound, the method including a step of incorporating at least one substance selected from the group consisting of polyanions and salts thereof, dextran, cyclodextrin, polyethylene glycol, and glycerol in the dispersion of the microparticles."

Patent Document 2 discloses an object of "improving particle sedimentation properties while maintaining high reagent sensitivity for a latex turbidimetric immunoassay reagent", and discloses that this object can be achieved by "a latex turbidimetric immunoassay reagent composed of a suspension of latex particles onto which an antigen or antibody has been supported, wherein the reagent contains from 7.5 to 15% by weight/volume of saccharides."

Patent Document 3 discloses, as an improved method for regulating an assay involving the interaction of two binding partners, "a method for determining an analyte in a sample within an interaction assay, the method including bringing the sample into contact with an interaction modulator, wherein the interaction modulator is an enhancer of the interaction assay at low analyte concentration and a retarder of the interaction assay at high analyte concentration, the interaction modulator is a copolymer of 4-styrenesulfonic acid and maleic acid (poly-(4-styrenesulfonic acid-co-maleic acid, PSSM) and/or polyacrylic acid (PAA), and the interaction assay is a homogeneous agglutination assay."

### Citation List

### Patent Documents

Patent Document 1
   Japanese Unexamined Patent Application, First Publication No. 2007-114129
Patent Document 2
   Japanese Unexamined Patent Application, First Publication No. Hei 11-051938
Patent Document 3
   Japanese Translation of Unexamined PCT Application No. 2020-507752

### Summary of Invention

### Problems to be solved by Invention

In the method for suppressing sedimentation of fine particles disclosed in Patent Document 1, a sedimentation suppression effect was confirmed only for metal colloid particles of 5 to 100 nm, and absolutely no mention is made regarding sedimentation suppression for latex particles in a latex reagent used in the LTIA method.

The fine particle sedimentation suppression method disclosed in Patent Document 2 is a technique which aims to increase the specific gravity and improve the dispersion properties of a particle suspension by incorporating saccharides including monosaccharides such as glucose and fructose, disaccharides such as sucrose and lactose, or oligosaccharides such as maltotriose within the particle suspension. However, investigations conducted by the inventors of the present invention clearly showed that if these types of saccharides are added to a latex reagent used in the LTIA method, then there is a possibility that the sensitivity of the detection reagent may deteriorate (Test Example 3).

In the method disclosed in Patent Document 3, the enhancer such as polyacrylic acid is not added to a solution containing latex particles. Accordingly, enhancers such as polyacrylic acid do not improve the on-board stability of latex reagents.

Until now, no technology has existed that can suppress the sedimentation of latex particles in a latex particle dispersion used in the LTIA method, without adversely affecting the reagent performance, such as reducing the sensitivity of the detection reagent.

The present invention has the objects of providing a latex particle dispersion, a reagent kit, a detection method, a sedimentation suppressing agent, and a method for suppressing sedimentation that exhibit improved on-board stability.

### Means to Solve the Problems

As a result of intensive investigation aimed at achieving the above objects, the inventors of the present invention discovered that in a detection reagent composed of a buffer solution and a latex particle dispersion that is used for detecting a target substance by latex turbidimetric immunoassay, by including at least one compound selected from the group consisting of hexametaphosphoric acid and salts thereof in the latex particle dispersion, sedimentation of the latex particles in the latex particle dispersion can be suppressed for a long period of time, and when applied to the detection of a target substance by latex turbidimetric immunoassay, the measurement sensitivity is at least maintained compared with a reagent not containing one of these added components, enabling the inventors to complete the present invention.

The present invention includes the following aspects.
[1] A latex particle dispersion containing latex particles and an aqueous medium, and also containing at least one compound selected from the group consisting of hexametaphosphoric acid and salts thereof.
[2] The latex particle dispersion according to [1], which is used as a latex particle dispersion in a detection reagent used in the detection of a target substance by latex turbidimetric immunoassay.
[3] The latex particle dispersion according to [2], wherein the detection reagent contains a buffer solution and the latex particle dispersion.
[4] The latex particle dispersion according to any one of [1] to [3], wherein the salts are alkali metal salts.
[5] The latex particle dispersion according to any one of [1] to [4], wherein the aqueous medium contains water.
[6] The latex particle dispersion according to any one of [1] to [5], wherein the latex particles are polymer particles derived from a monomer component that includes a monomer having a styrene structure or a vinylnaphthalene structure.
[7] The latex particle dispersion according to any one of [1] to [6], wherein the latex particles are latex particles onto which a substance capable of binding to a target substance has been supported.
[8] The latex particle dispersion according to [7], wherein the substance capable of binding to a target substance is an antigen or antibody for the target substance.
[9] The latex particle dispersion according to any one of [1] to [8], wherein the average particle size of the latex particles is within a range from 50 to 1,000 nm.
[10] The latex particle dispersion according to any one of [1] to [9], wherein the latex particles are included in an amount within a range from 0.001 to 0.20% by mass relative to the total mass of the latex particle dispersion.
[11] A reagent kit used in the detection of a target substance in a specimen by latex turbidimetric immunoassay, wherein
   the reagent kit contains the latex particle dispersion according to any one of [1] to [10].
[12] A method for detecting a target substance in a specimen by latex turbidimetric immunoassay, the method including:
   a step of bringing the latex particle dispersion according to any one of [1] to [10] and the specimen into contact.
[13] A sedimentation suppressing agent for latex particles within a latex particle dispersion included in a detection reagent containing a buffer solution and the latex particle dispersion that is used in the detection of a target substance by latex turbidimetric immunoassay, wherein
   the sedimentation suppressing agent contains at least one compound selected from the group consisting of hexametaphosphoric acid and salts thereof as the active ingredient.
[14] The sedimentation suppressing agent according to [13], wherein the salts are alkali metal salts.
[15] A method for suppressing sedimentation of latex particles onto which a substance capable of binding to a target substance has been supported in a latex turbidimetric immunoassay, the method including
   a step of bringing the latex particles and at least one compound selected from the group consisting of hexametaphosphoric acid and salts thereof into contact within an aqueous medium.
[16] The method for suppressing sedimentation according to [15], wherein the salts are alkali metal salts.
[17] A method for storing latex particles for latex turbidimetric immunoassay, the method including:
   a step of bringing the latex particles and at least one compound selected from the group consisting of hexametaphosphoric acid and salts thereof into contact within an aqueous medium.
[18] A method for storing a latex particle dispersion for latex turbidimetric immunoassay, the method including:
   a step of bringing the latex particles and at least one compound selected from the group consisting of hexametaphosphoric acid and salts thereof into contact within an aqueous medium, wherein
   the amount of latex particles contained in the supernatant of the latex particle dispersion after 7 days of storage is at least 80% of the amount prior to storage.

### Effects of Invention

The present invention is able to provide a latex particle dispersion having improved on-board stability, a reagent kit, a detection method, a sedimentation suppressing agent, and a method for suppressing sedimentation.

### Brief Description of the Drawings

[FIG. 1] FIG. 1 is a graph illustrating the latex particle content ratio before and after inversion mixing in Test Example 1.
[FIG. 2] FIG. 2 is a graph illustrating the latex particle content ratio before and after inversion mixing in Test Example 2.

### Description of Embodiments

In this description and in the claims, when a numerical range is described using the expression "a to b", the numerical values on either side of the "to" are included within that numerical range.

### [Particle Dispersion]

A latex particle dispersion of the present invention contains latex particles and an aqueous medium, and also contains at least one compound selected from the group consisting of hexametaphosphoric acid and salts thereof.

By including the above compound with the latex particles, the dispersibility of the latex particles within the aqueous medium is improved, and as a result, an improvement in the on-board stability can be achieved.

Among the hexametaphosphoric acid and salts thereof that can be used in the latex particle dispersion of the present invention, the salts are preferably water-soluble salts. There are no particular limitations on the salts, but examples include the ammonium salt and alkali metal salts, and alkali metal salts are preferred.

Examples of the alkali metal salts include lithium (Li), sodium (Na), potassium (K), rubidium (Rb), cesium (Cs) and francium (Fr) salts. The alkali metal salt is preferably at least one salt selected from the group consisting of the sodium salt and the potassium salt. Moreover, the sodium salt is most preferable, namely, sodium hexametaphosphate (CAS RN [10124-56-8]) is the most preferred. A single alkali metal salt may be used alone, or a combination of two or more alkali metal salts may be used.

There are no particular limitations on the amount of the above compound in the latex particle dispersion of the present invention, but relative to the total mass of the latex particle dispersion, the amount is preferably within a range from 0.001 to 10.0% by mass, more preferably from 0.001 to 5.0% by mass, even more preferably from 0.005 to 2.0% by mass, and still more preferably from 0.01 to 1.0% by mass.

An amount of the above compound in the latex particle dispersion of the present invention within the range from 0.001 to 10.0% by mass relative to the total mass of the latex particle dispersion causes no deterioration in the measurement sensitivity, meaning the dispersibility of the latex particle dispersion can be improved without adversely affecting the target substance detection precision.

There are no particular limitations on the pH of the latex particle dispersion of the present invention, but the pH is preferably within a range from pH 4.0 to pH 10.0, more preferably from pH 5.0 to pH 9.0, and even more preferably from pH 6.0 to pH 8.0.

Adjustment and measurement of the pH may be conducted using techniques commonly known to those having ordinary knowledge in the technical field to which the invention belongs. For example, the pH of the latex particle dispersion of the present invention can be adjusted by adding a base such as sodium hydroxide or an acid such as hydrochloric acid.

The latex particle dispersion of the present invention is used as a latex particle dispersion included in a detection reagent used in the detection of a target substance by latex turbidimetric immunoassay. The detection reagent is generally composed of two reagents, but may have three or more reagents. In this description, any solution containing added latex particles may be termed a "latex particle dispersion". In those cases where the detection reagent is composed of two reagents, the detection reagent is generally composed of a buffer solution (the first reagent) and the latex particle dispersion (the second reagent). The detection reagent may also have a composition in which latex particles are added to the first reagent, or a composition in which latex particles are added to both the first reagent and the second reagent. The latex particle dispersion of the present invention may be the first reagent, the second reagent, or the third or greater reagent used in the latex turbidimetric immunoassay. The latex particle dispersion of the present invention is preferably the latex particle dispersion included in a detection reagent composed of a buffer solution and the latex particle dispersion.

The latex particle dispersion of the present invention can be used as a reagent for detecting a target substance by latex turbidimetric immunoassay, and can be combined with other reagent compositions to form a kit. Examples of these reagents include typical external diagnostic reagents. The composition of the reagent kit is described below. A reagent used in a latex turbidimetric immunoassay is sometimes referred to as a turbidimetric immunoassay reagent.

### (Aqueous Medium)

The aqueous medium used in the latex particle dispersion of the present invention may be any medium capable of dispersing the latex particles, and among such mediums, mediums containing water are preferred. There are no particular limitations on the amount of water, provided the latex particles are able to be dispersed, but the amount of water, expressed relative to the total mass of the aqueous medium, may be, for example, within a range from 40 to 100% by mass, from 60 to 100% by mass, from 80 to 100% by mass, or from 90 to 100% by mass.

Further, the aqueous medium may be a buffer solution such as a Good's buffer solution, TRIS buffer solution, phosphate buffer solution, glycine buffer solution, ammonia buffer solution, citrate buffer solution, acetate buffer solution, or succinate buffer solution.

Besides the water and buffer solution described above, the aqueous medium may also contain organic solvents that are miscible with water, including lower alcohols such as methanol, ethanol and isopropanol, and dimethyl sulfoxide and dimethylformamide.

Furthermore, in addition to the water and buffer solution described above, the aqueous medium may also contain proteins such as bovine serum albumin and casein, as well as amino acids, saccharides, surfactants, antifoaming agents, and preservatives and the like.

The amount of the aqueous medium, relative to the total mass of the latex particle dispersion, may be, for example, within a range from 50.00 to 99.99% by mass, or from 70.00 to 99.99% by mass.

### (Latex Particles)

In the latex particle dispersion of the present invention, there are no particular limitations on the latex particles, provided they are the type of latex particles typically used as an immunoassay reagent, and the latex particles may contain a fluorescent dye or a magnetic substance. The latex particles can be obtained by polymerizing or copolymerizing any of various monomers. Examples of the monomers include polymerizable unsaturated aromatics, including polymerizable monomers having a phenyl group such as styrene, α-methylstyrene, o-methylstyrene, p-methylstyrene, p-chlorostyrene, 4-vinylbenzoic acid, divinylbenzene and vinyltoluene, polymerizable monomers having a phenyl group and a sulfonate group such as styrenesulfonates, divinylbenzenesulfonates, o-methylstyrenesulfonates and p-methylstyrenesulfonates, and polymerizable monomers having a naphthyl group such as 1-vinylnaphthalene, 2-vinylnaphthalene, α-naphthyl (meth)acrylate and β-naphthyl (meth)acrylate; polymerizable unsaturated carboxylic acids such as (meth)acrylic acid, itaconic acid, maleic acid and fumaric acid; polymerizable unsaturated carboxylic acid esters such as methyl (meth)acrylate, ethyl (meth)acrylate, n-butyl (meth)acrylate, 2-hydroxyethyl (meth)acrylate, glycidyl (meth)acrylate, ethylene glycol di(meth)acrylate ester and tribromophenyl (meth)acrylate; and unsaturated carboxylic acid amides, polymerizable unsaturated nitriles, halogenated vinyl compounds and conjugated dienes such as (meth)acrylonitrile, (meth)acrolein, (meth)acrylamide, N-methylol (meth)acrylamide, methylene bis(meth)acrylamide, butadiene, isoprene, vinyl acetate, vinylpyridine, N-vinylpyrrolidone, vinyl chloride, vinylidene chloride and vinyl bromide. Among the various possibilities, polymer particles derived from a monomer component containing a monomer having a styrene structure or a vinylnaphthalene structure are preferred. Here, unless specifically stated otherwise, the expression "polymer particles derived from a monomer component" means polymer particles obtained by polymerizing or copolymerizing the monomer component.

These monomers may be selected appropriately in accordance with the required surface characteristics and specific gravity and the like, and either a single monomer may be used alone, or a combination of two or more monomers may be used.

There are no particular limitations on the synthetic polymer that constitutes the latex particles of the present invention, and examples include polystyrene, styrene-styrenesulfonate copolymers, methacrylic acid polymers, acrylic acid polymers, itaconic acid polymers, and styrene-hydrophilic carboxy monomer copolymers such as styrene-(meth)acrylic acid copolymers, styrene-acrylic acid copolymers and styrene-itaconic acid copolymers. Among these, styrene-methacrylic acid copolymers, styrene-itaconic acid copolymers, styrene, and styrene-styrenesulfonate copolymers are preferred.

The amount of the latex particles, relative to the total mass of the latex particle dispersion, is preferably within a range from 0.001 to 0.20% by mass.

The average particle size of the latex particles in the present invention may be any size that enables a sensitivity difference to be obtained for the measurement target in the measurement range, and is preferably within a range from 50 nm to 1,000 nm. When the average particle size of the latex particles is large, although sensitivity improves, the particles tend to be more prone to sedimentation. Accordingly, even for particles which have conventionally been difficult to use in measurement reagents, by employing the latex particle dispersion of the present invention, sedimentation becomes less likely even when the particle size is large. Moreover, even in the case of latex particles having a mid-size or small average particle size, by adding the component of the present invention, a state that is resistant to sedimentation of the particles for longer periods of time can be achieved. Accordingly, the effects of the present invention can be realized regardless of the average particle size of the latex particles.

Although there are no particular limitations on the average particle size of the latex particles, the average particle size is preferably at least 50 nm, more preferably at least 80 nm, and even more preferably 100 nm or greater.

Further, the measurable concentration range can be further expanded by using latex particles having two or more different average particle sizes.

The average particle size of the latex particles can be analyzed using the laser diffraction/scattering method (LS method), the Coulter principle, the dynamic light scattering photon correlation method, or using an electronic microscope or the like. In the present invention, the average particle size means the volume-based average particle size, and may be a value obtained by a laser diffraction/scattering particle size distribution measurement device based on the laser diffraction/scattering method (LS method). For example, an LS 13 320 analyzer manufactured by Beckman Coulter, Inc. may be used as the laser diffraction/scattering particle size distribution measurement device, and the measurement parameters may be set appropriately in accordance with the solvent used and the composition of the measurement target latex particles. The validity of the measurement parameters can be confirmed by ensuring that the measured particle sizes for standard polystyrene particles (that have been certified by a third party) available commercially from Beckman Coulter, Inc. and the like fall within the specifications prescribed by the manufacturer.

### (Substance Capable of Binding to Target Substance)

In the present invention, examples of the substances capable of binding to the target substance include proteins, peptides, amino acids, lipids, sugars, nucleic acids and haptens, and although there are no particular limitations on the size of the molecular weight or whether the derivation of the substance is natural or synthetic, specific examples include antibodies or antigens that can be used in immunoassays that utilize an immunological reaction.

The antibodies may be polyclonal antibodies or monoclonal antibodies. Monoclonal antibodies are more preferred.

Examples of antibodies that can be used in the present invention include not only antibody entire molecules, but also functional fragments of antibodies that exhibit antigen-antibody reaction activity. In addition to antibodies obtained through the immunization of common animals (mice, goats, and sheep and the like), antibodies (such as chimeric antibodies, humanized antibodies, or fully humanized antibodies or the like) in which the amino acid sequence has been altered using gene recombination technology to the amino acid sequence of an animal species different from the animal immunized with the immunogen (the target substance) may also be used. Examples of functional fragments of antibodies include fragments that exhibit antigen-antibody reaction activity such as F(ab')₂, Fab' and single-chain antibodies (scFv), VHH (variable domain of the heavy chain of heavy-chain antibody) antibodies, and IgNAR (new antigen receptor) antibodies. These functional fragments of antibodies can be produced by treating antibodies obtained in the manner described above with a protease (such as pepsin or papain or the like).

In the present invention, the substance supported on the latex particles that is capable of binding to the target substance can be immobilized and supported on the latex particles using a conventional method such as a physical adsorption method, a chemical binding method, or a combination of these methods.

In the case of a physical adsorption method, the adsorption may be conducted in accordance with a conventional method, by mixing and contacting the substance capable of binding to the target substance and the latex particles in a solution such as a buffer solution, or by bringing the substance capable of binding to the target substance dissolved in a buffer solution or the like into contact with the carrier.

In the case of a chemical binding method, the substance capable of binding to the target substance and the latex particles may be mixed and brought into contact with a divalent crosslinking reagent such as glutaraldehyde, a carbodiimide, an imide ester or a maleimide, in accordance with a known method disclosed in "Clinical Pathology Extra Supplementary Special Feature No. 53: Immunoassay for Clinical Testing - Technology and Applications" edited by the Japanese Society of Clinical Pathology, published by Clinical Pathology Publishing Association, 1983; or "New Biochemical Testing Course 1: Protein IV" edited by the Japanese Biochemical Society, published by Tokyo Kagaku Dojin, 1991, thereby reacting the amino groups, carboxyl groups, thiol groups, aldehyde groups, or hydroxyl groups or the like of the substance capable of binding to the target substance and the latex particles with the divalent crosslinking reagent.

If a treatment is required to suppress spontaneous agglutination of the latex particles or non-specific reactions or the like, then a carrier blocking treatment (masking treatment) may be conducted by treating the surface of the latex particles using a known method such as surface coating by contact with a protein such as bovine serum albumin (BSA), casein, gelatin, egg white albumin or a salt thereof, a surfactant, or skim milk powder or the like. Further, as described in Japanese Unexamined Patent Application, First Publication No. Hei 11-337551, latex particles having an antigen or antibody supported thereon that does not undergo an immunological reaction with the target substance may also be added to the detection reagent. These types of particles are generally added to the first reagent with the purpose of suppressing non-specific reactions.

One aspect of the present invention relates to the use of a latex particle dispersion in a detection reagent used for detecting a target substance by latex turbidimetric immunoassay.

Further, another aspect relates to the use of a latex particle dispersion for detecting a target substance by latex turbidimetric immunoassay.

Further, yet another aspect relates to the use of a latex particle dispersion for producing a detection reagent used in detecting a target substance by latex turbidimetric immunoassay.

Moreover, yet another aspect relates to the use of latex particles, an aqueous medium, and at least one compound selected from the group consisting of hexametaphosphoric acid and salts thereof for producing a latex particle dispersion.

### [Reagent Kit]

A reagent kit for use in detecting a target substance in a specimen by latex turbidimetric immunoassay contains at least the latex particle dispersion of the present invention described above. Examples of the composition of the kit are typically two-reagent compositions that include a particle dispersion containing particles onto which a substance capable of binding to the target substance has been supported (the second reagent), and a first reagent containing a buffer solution. Examples of the buffer solution used in the first reagent include Good's buffer solutions, TRIS buffer solutions, phosphate buffer solutions, glycine buffer solutions, ammonia buffer solutions, citrate buffer solutions, acetate buffer solutions, and succinate buffer solutions. Further, besides the buffer solution, the first reagent may also contain components for suppressing non-specific reactions, agglutination promoters, antifoaming agents, and/or preservatives or the like. Furthermore, other components that may be included in the kit besides the first reagent and the second reagent include a sample dilution liquid, a sample extraction liquid, an instruction manual, and sample collection tools (such as a collection pipette, syringe, cotton swab, and/or filtration filter or the like).

### [Detection Method]

A method for detecting a target substance in a specimen by latex turbidimetric immunoassay (LTIA) according to the present invention is conducted by bringing the latex particle dispersion described above and the specimen into contact.

Methods for measuring a target substance by LTIA can be broadly classified into two types.

The first type describes methods in which latex particles onto which a substance capable of binding to the target substance has been supported are reacted with the target substance to form a sandwich-type immunoconjugate, and the target substance is measured based on the degree of agglutination of the latex particles that accompanies the formation of the immunoconjugate.

The second type describes methods in which a plurality of target substances or analogs thereof (including fragments thereof), or a protein or the like onto which the plurality of target substances or analogs have been immobilized, is added to the reagent, thereby causing competition between these substances and the target substance in the sample and inhibiting the formation of an immunoconjugate between the target substance contained in the reagent and the latex particles onto which a substance capable of binding to the target substance has been supported, and the target substance (antigen) is then measured based on the degree of inhibition of agglutination of the latex particles that accompanies the inhibition of immunoconjugate formation.

The target substance and the substance capable of binding to the target substance may be any of various substances selected in accordance with the intended purpose. For example, if the target substance is an antigen, then an antibody such as a polyclonal antibody or monoclonal antibody (including gene recombinant antibodies, and functional fragments of various antibodies) may be selected as the substance capable of binding to the target substance. If the target substance is an antibody, then an antigen such as a natural antigen or gene recombinant antigen may be selected as the substance capable of binding to the target substance.

The present invention may be used in any of the methods described above, but specifically, may be used in a method having the following steps.
(1) A step of mixing a target substance within a specimen with a buffer solution or the like.
(2) A step, conducted after step (1), of adding a latex particle dispersion containing latex particles onto which a substance capable of binding to the target substance has been supported to the obtained mixed solution, thereby bringing the latex particle dispersion into contact with the specimen.
(3) A step of optically detecting the degree of agglutination of the latex particles in the solution.

The latex particles onto which a substance capable of binding to the target substance has been supported used in step (2) may be composed of one type, two types, or three or more types of particles. Further, a portion of the latex particles may be incorporated in the buffer solution of step (1). Step (3) may be started when step (2) is started, or when step (1) is started.

In the present invention, examples of the method used for optically detecting the degree of agglutination of the latex particles include methods (such as endpoint methods and rate methods) for measuring the scattered light intensity, the absorbance, or the transmitted light intensity using optical equipment. The measured value for the absorbance or the like obtained by measuring the sample is compared with the measured values for the absorbance or the like obtained upon measurement of standard substances (samples with known concentrations of the target substance), enabling calculation of the concentration (quantitative value) of the target substance contained in the sample. The measurement of the absorbance or the like of the transmitted light or scattered light or the like may be a one-wavelength measurement or a two-wavelength measurement (using the difference or ratio between the two wavelength values). The measurement wavelength is generally selected from within a range from 500 nm to 900 nm.

In the present invention, the measurement of the target substance in a specimen may be conducted manually, or may be conducted using a device such as a measurement device, and is preferably conducted using a device. The measurement device may be a general-purpose automatic analyzer or a special-purpose measurement device (dedicated device).

### [Sedimentation Suppressing Agent]

A sedimentation suppressing agent for the latex particles in a latex particle dispersion included in a detection reagent containing a buffer solution and the latex particle dispersion that is used in the detection of a target substance by latex turbidimetric immunoassay according to the present invention has at least one compound selected from the group consisting of hexametaphosphoric acid and salts thereof as an active ingredient.

One aspect of the present invention relates to the use of at least one compound selected from the group consisting of hexametaphosphoric acid and salts thereof for suppressing the sedimentation of latex particles in a latex particle dispersion included in a detection reagent containing a buffer solution and the latex particle dispersion that is used in the detection of a target substance by latex turbidimetric immunoassay.

Another aspect relates to the use of at least one compound selected from the group consisting of hexametaphosphoric acid and salts thereof for producing a sedimentation suppressing agent for the latex particles in a latex particle dispersion included in a detection reagent containing a buffer solution and the latex particle dispersion that is used in the detection of a target substance by latex turbidimetric immunoassay.

Further, yet another aspect relates to the use of at least one compound selected from the group consisting of hexametaphosphoric acid and salts thereof for suppressing the sedimentation of the latex particles in a latex particle dispersion.

Moreover, yet another aspect relates to the use of at least one compound selected from the group consisting of hexametaphosphoric acid and salts thereof for producing a sedimentation suppressing agent for the latex particles in a latex particle dispersion.

The type and amount of the above active ingredient in the latex particle dispersion may be the same as that described above in relation to the latex particle dispersion of the present invention.

### [Method for Suppressing Sedimentation]

A method for suppressing sedimentation of latex particles in a latex turbidimetric immunoassay according to the present invention is a method that includes a step of bringing the latex particles and at least one compound selected from the group consisting of hexametaphosphoric acid and salts thereof into contact within an aqueous medium. Although the details of the mechanism are not entirely clear, it is thought that by bringing the above compound and the latex particles into contact within an aqueous medium, sedimentation of the latex particles can be suppressed.

One aspect of the present invention is a method for suppressing sedimentation of latex particles that includes a step of mixing a latex particle dispersion and at least one compound selected from the group consisting of hexametaphosphoric acid and salts thereof.

Further, another aspect is a method for suppressing sedimentation of latex particles in which the above mixing is conducted within an aqueous medium.

In the present invention, the expression that the "latex particles sediment" includes both those cases where the particles themselves sediment, and those cases where the particles adsorb to one another or agglutinate, resulting in an increase in particle size and subsequent sedimentation. Moreover, in the present invention, the expression "suppressing the sedimentation of latex particles" includes cases of suppression of sedimentation of the particles themselves, cases of suppression of the adsorption or agglutination of particle to one another resulting in an increase in particle size and sedimentation, and cases of suppression of both these forms of sedimentation.

In the present invention, the existence or absence of a latex particle sedimentation suppression effect is evaluated, for example, in the manner described below in the examples, by adding a test substance additive to the latex particle dispersion, and then in those cases where the degree of particle sedimentation improves compared with the case where the additive is not added, deeming that the additive has a sedimentation suppression effect. The degree of particle sedimentation can be evaluated by comparing the absorbance of the latex particle dispersion at 600 nm or the like prior to mixing, and the absorbance at the same wavelength after mixing. For example, the container in which the latex particle dispersion is stored is left to stand for 7 days or 15 days, a solution sample is then extracted from the upper portion of the dispersion, and the absorbance is measured. In those cases where the absorbance after mixing compared with the absorbance prior to mixing is, for example, 80.0% or greater, the additive is evaluated as having a sedimentation suppression effect.

### [Storage Method]

A storage method for a latex particle dispersion in a latex turbidimetric immunoassay method according to the present invention is a method that includes a step of conducting storage in a state where at least one compound selected from the group consisting of hexametaphosphoric acid and salts thereof has been added to the latex particle dispersion. Although the details of the mechanism are not entirely clear, by bringing the above compound and the latex particles into contact within an aqueous medium, sedimentation of the latex particles is suppressed. As a result, it is thought that the latex particles in the latex particle dispersion can be stored (preserved) for long periods without sedimenting.

One aspect of the present invention relates to a storage method for a latex particle dispersion in a latex turbidimetric immunoassay, wherein the method includes a step of mixing the latex particle dispersion and at least one compound selected from the group consisting of hexametaphosphoric acid and salts thereof.

In the present invention, an evaluation as to whether or not stable storage is possible without sedimentation of the latex particles in a latex particle dispersion can be made, for example, in the manner described below in the examples, by adding a test substance additive to the latex particle dispersion, and then in those cases where the degree of particle sedimentation improves compared with the case where the additive is not added, deeming that stable storage is possible. The degree of particle sedimentation can be evaluated by comparing the absorbance of the latex particle dispersion at 600 nm or the like prior to mixing, and the absorbance at the same wavelength after mixing. For example, the container in which the latex particle dispersion is stored is left to stand for 7 days, a solution sample is then extracted from the upper portion of the dispersion, and the absorbance is measured. In those cases where the absorbance after mixing compared with the absorbance prior to mixing is, for example, 80.0% or greater, a stable storage effect is deemed to have been achieved.

### [Specimen]

In the present invention, examples of the specimen containing the target substance include samples obtained from living bodies, such as human or animal blood, serum, plasma, culture supernatants, urine, cerebrospinal fluid, saliva, sweat, ascites, or cell or tissue extracts.

Further, in the present invention, the specimen includes not only the samples themselves obtained from living bodies, but also samples that have undergone some form of pretreatment such as dilution, purification, extraction or filtration or the like. Examples of blood specimens include whole blood, red blood cells, blood plasma, and blood serum and the like, as well as plasma specimens collected in a blood-collecting vessel to which an anticoagulant has been added.

### [Target Substance]

In the present invention, examples of the target substance include proteins, peptides, amino acids, lipids, sugars, nucleic acids, and haptens, but there are no particular limitations on the target substance, provided it is a substance that can be theoretically measured. Specific examples include CRP (C-reactive protein), Lp(a), MMP3 (matrix metalloproteinase 3), antiphospholipid antibodies, type IV collagen, PSA, BNP (brain natriuretic peptide), insulin, albumin, cystatin C, RF (rheumatoid factor), KL-6, procalcitonin (PCT), FDP, D-dimer, SF (soluble fibrin), TAT (thrombin-antithrombin III complex), PAI-1, phenytoin, phenobarbital, carbamazepine, valproic acid, theophylline, TARC (thymus and activation-regulated chemokine), and sIL-2R (soluble interleukin-2 receptor).

### Examples

The present invention is described below in further detail using a series of examples, but the present invention is not limited to only the following examples.

### [Test Example 1: Latex Particle Sedimentation Evaluation: Procalcitonin Measurement Reagent]

Various additives were each added to a latex particle dispersion (LTIA measurement reagent second reagent) and stored, and the sedimentation of the latex particles in the solution was evaluated.

### <Comparative Example 1>

The test method and evaluation method were as follows.

### (1) Preparation of LTIA Measurement Reagent Second Reagent

- 5 mM MOPS-NaOH (pH 7.0)
- Anti-human procalcitonin monoclonal antibody-sensitized latex (2 types)

The anti-human procalcitonin monoclonal antibodies were acquired using a method known to those skilled in the art, using a commercially available procalcitonin antigen (*). Moreover, combinations of monoclonal antibodies capable of sandwich measurement of the procalcitonin antigen were selected using methods known to those skilled in the art. The anti-human procalcitonin monoclonal antibody-sensitized latexes were prepared with reference to the method disclosed in Japanese Unexamined Patent Application, First Publication No. 2017-181377. Specifically, to a 1.0% latex solution (5 mM Tris buffer solution (hereinafter referred to as Tris-HCl or simply Tris) (pH 8.5)) with an average particle size of 0.3 µm was added an equivalent volume of an anti-human procalcitonin monoclonal antibody solution diluted to 0.36 mg/mL with 5 mM Tris-HCl (pH 8.5), and the resulting mixture was stirred at 4°C for two hours. An equal volume of 5 mM Tris-HCl (pH 8.5) containing 0.5% BSA was then added and stirred at 4°C for one hour to prepare an anti-human procalcitonin monoclonal antibody-sensitized latex particle solution. This latex particle solution was diluted with 5 mM MOPS-NaOH (pH 7.0) to achieve an absorbance of about 5.5 OD at 600 nm, and the resulting solution was used as the second reagent of an LTIA measurement reagent.
(*) Commercially available antigen: Recombinant human procalcitonin, manufactured by HyTest Ltd.

### (2) Storage Conditions

Two mL samples of the above second reagent were dispensed into two glass test tubes, and left to stand in a refrigerator (2 to 8°C) for 7 days.

### (3) Evaluation Method

One hundred µL samples were extracted from the liquid surface of the two glass test tubes, with one test tube being mixed by inversion prior to sampling while the other was kept still, each sample was then mixed with 900 µL (a 10-fold dilution) of 5 mM MOPS-NaOH (pH 7.0), and the absorbance at 600 nm and 800 nm was measured. The ratio of the absorbance at 600 nm relative to the absorbance at 800 nm (the 600/800 ratio) was calculated as an indicator representing the dispersibility of particles in the solution. The absorbance ratio of the absorbance values at 600 nm (prior to inversion mixing) / (after inversion mixing) was calculated as the latex particle content ratio (%) at the liquid surface.

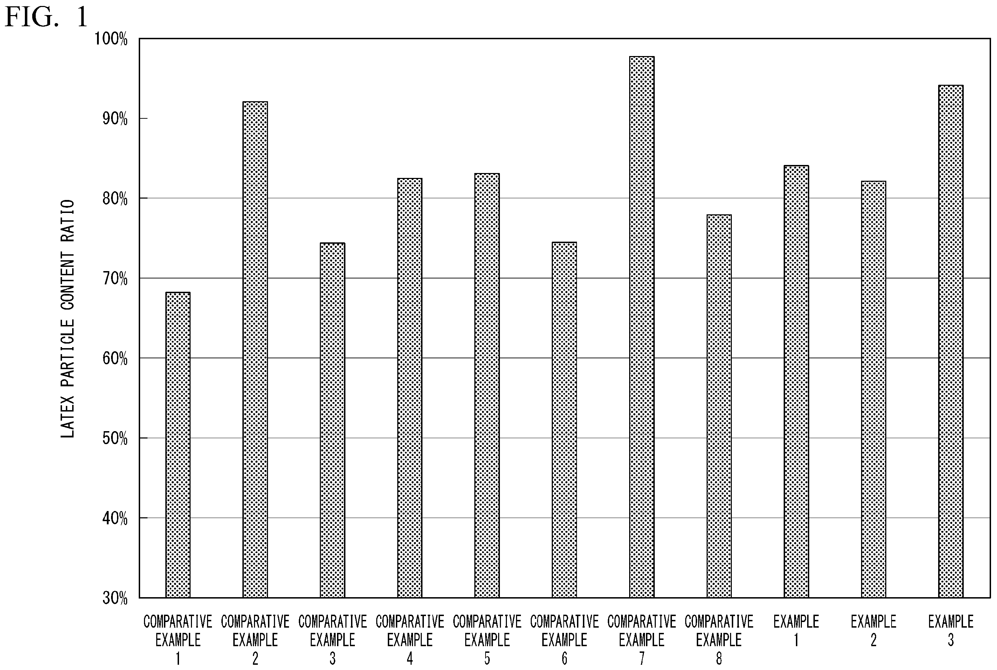

The measurement results are shown in Table 1. The latex particle content ratio prior to and after inversion mixing is shown in the graph of FIG. 1. The measured absorbance values at 600 nm and 800 nm were multiplied by 10, and the resulting absorbance of the raw solution was recorded in Table 1.

### <Comparative Examples 2 to 8, Examples 1 to 3>

With the exception of adding the component shown in the column labeled "Additive name" in Table 1 to the LTIA measurement reagent second reagent described in Comparative Example 1 in an amount sufficient to obtain the final concentration shown in the column labeled "Final concentration", measurements were conducted in the same manner as described above. The measurement results are shown in Table 1. The products listed below were used as additives.
- Sucrose, polyacrylic acid 5000, polyacrylic acid 25000, polyacrylic acid 1000000, sodium polyacrylate 22000-70000, sodium polyphosphate, sodium hexametaphosphate: manufactured by FUJIFILM Wako Pure Chemical Corporation. Numbers appended to the polyacrylic acid products indicate polymerization degrees.
- PEO-3: manufactured by Sumitomo Seika Chemicals Co., Ltd., molecular weight: 600,000 to 1,100,000.

**[Table 1]**

| | Additive | | Prior to inversion mixing | | | After inversion mixing | | | Latex particle content ratio | Sedimentation suppression function | Degree of reagent foaming |
|---|---|---|---|---|---|---|---|---|---|---|---|
| | Name of additive | Final concentration | Absorbance (600 nm) | Absorbance (800 nm) | 600/800 ratio | Absorbance (600 nm) | Absorbance (800 nm) | 600/800 ratio | | | |
| Comparative Example 1 | no additive | - | 4.544 | 2.094 | 2.17 | 6.663 | 3.091 | 2.16 | 68.2% | - | A |
| Comparative Example 2 | sucrose | 5% | 5.314 | 2.445 | 2.17 | 5.771 | 2.679 | 2.15 | 92.1% | A | not tested |
| Comparative Example 3 | polyacrylic acid 5000 | 0.01% | 4.973 | 2.288 | 2.17 | 6.686 | 3.103 | 2.15 | 74.4% | B | B |
| Comparative Example 4 | polyacrylic acid 25000 | 0.01% | 5.446 | 2.507 | 2.17 | 6.603 | 3.067 | 2.15 | 82.5% | A | B |
| Comparative Example 5 | polyacrylic acid 1000000 | 0.25% | 5.394 | 2.494 | 2.16 | 6.492 | 3.060 | 2.12 | 83.1% | A | B |
| Comparative Example 6 | PEO-3 | 0.1% | 4.719 | 2.173 | 2.17 | 6.337 | 2.949 | 2.15 | 74.5% | B | B |
| Comparative Example 7 | Na polyacrylate 22000-70000 | 0.1% | 5.791 | 2.678 | 2.16 | 5.926 | 2.750 | 2.15 | 97.7% | A | B |
| Comparative Example 8 | sodium polyphosphate | 0.01% | 5.231 | 2.413 | 2.17 | 6.714 | 3.122 | 2.15 | 77.9% | B | not tested |
| Example 1 | sodium hexametaphosphate | 0.005% | 5.666 | 2.632 | 2.15 | 6.738 | 3.134 | 2.15 | 84.1% | A | A |
| Example 2 | | 0.01% | 5.474 | 2.532 | 2.16 | 6.664 | 3.103 | 2.15 | 82.1% | A | A |
| Example 3 | | 0.02% | 6.204 | 2.860 | 2.17 | 6.590 | 3.065 | 2.15 | 94.1% | A | A |

### (4) Results and Observations

The ratio of the absorbance at 600 nm relative to the absorbance at 800 nm (the 600/800 ratio) indicates the dispersibility of the latex particles in the reagent. Based on the 600/800 ratio for Comparative Example 1 (no additive) after inversion mixing (in this case: 2.17), conditions under which this value did not decrease by 0.10 or more were evaluated as exhibiting favorable dispersibility. This evaluation method was also used in Test Example 2. Comparative Examples 2 to 8 and Examples 1 to 3 all exhibited favorable dispersibility.

The absorbance ratio of the absorbance values at 600 nm (prior to inversion mixing) / (after inversion mixing) was calculated as the latex particle content ratio (%) at the liquid surface. A smaller value for this ratio indicates particles more prone to sedimentation, whereas a value closer to 100% was evaluated as indicating a higher particle sedimentation suppression effect for the additive. In this investigation, conditions which yielded a content ratio of 80% or higher were evaluated as having a particle sedimentation suppression function, and were recorded as "A" in the column labeled "Sedimentation suppression function" in Table 1. When the content ratio was less than 80%, there was deemed to be no particle sedimentation suppression function, and a "B" was recorded in the "Sedimentation suppression function" column in Table 1. Evaluation of the sedimentation suppression function for Comparative Example 1 was recorded as "-". Further, the degree of foaming of each LTIA measurement reagent second reagent was also evaluated and recorded in the column entitled "Degree of reagent foaming", with a "B" recorded when the reagent was clearly more prone to foaming compared with Comparative Example 1, and an "A" recorded when the degree of foaming was similar to that of Comparative Example 1. Further, examples in which the degree of foaming was not evaluated were recorded as "not tested". Reagents having a similar degree of foaming to Comparative Example 1 exhibited favorable handling properties, and were less prone to weighing errors when weighing out required amounts with a pipette or reagent probe of an automatic analyzer, and were consequently preferred.

The sucrose of Comparative Example 2 exhibited a sedimentation suppression function, but caused a deterioration in the measurement sensitivity (see Test Example 3 below), and was therefore not a preferred component.

The components of Comparative Examples 3 to 8 either exhibited little sedimentation suppression function, and/or if they exhibited a sedimentation suppression function, the reagent containing the added component tended to be prone to foaming, meaning none of these components was desirable.

Only the sodium hexametaphosphate used in Examples 1 to 3 exhibited a superior sedimentation suppression function and had a degree of foaming for the reagent containing this component that was similar to that of Comparative Example 1, meaning this compound exhibited the most superior properties.

### [Test Example 2: Latex Particle Sedimentation Evaluation: TARC Measurement Reagent]

Test Example 1 revealed that sodium hexametaphosphate had a superior sedimentation suppression function, and that a reagent containing added sodium hexametaphosphate had a degree of foaming similar to that of Comparative Example 1, indicating favorable handling properties. Next, compounds having a similar structure to sodium hexametaphosphate were added to latex particle dispersions (LTIA measurement reagent second reagents) and stored, and sedimentation of the latex particles in the solution was evaluated.

### <Comparative Example 9: No Additive>

The test method and evaluation method were as follows. Further, the reagent compositions are also described below.

### (1) Preparation of LTIA Measurement Reagent Second Reagent

- 5 mM MOPS-NaOH (pH 7.0)
- Anti-human TARC monoclonal antibody-sensitized latex (2 types)

The anti-human TARC monoclonal antibodies were acquired using a method known to those skilled in the art, using a commercially available TARC antigen (**). Moreover, combinations of monoclonal antibodies capable of sandwich measurement of the TARC antigen were selected using methods known to those skilled in the art. The anti-human TARC monoclonal antibody-sensitized latexes were prepared with reference to the method disclosed in Japanese Unexamined Patent Application, First Publication No. 2017-181377. Specifically, to a 1.0% latex solution (5 mM Tris buffer solution (hereinafter referred to as Tris-HCl or simply Tris) (pH 8.5)) with an average particle size of 0.3 µm was added an equivalent volume of an anti-human TARC monoclonal antibody solution diluted to 0.36 mg/mL with 5 mM Tris-HCl (pH 8.5), and the resulting mixture was stirred at 4°C for two hours. Subsequently, an equal volume of 5 mM Tris-HCl (pH 8.5) containing 0.5% BSA was added and stirred at 4°C for one hour to prepare an anti-human TARC monoclonal antibody-sensitized latex particle solution. This latex particle solution was diluted with 5 mM MOPS-NaOH (pH 7.0) to achieve an absorbance of about 5.5 OD at 600 nm, and the resulting solution was used as the second reagent of an LTIA measurement reagent.
(**) Commercially available antigens: CCL17, thymus and activation regulated chemokine (Shenandoah Biotechnology, Inc.), CCL17/TARC, Human (Lifespan Biosciences, Inc.), Human TARC (CCL17) (Abeomics, Inc.) and the like.

### (2) Storage Conditions

Two mL samples of the above second reagent were dispensed into two glass test tubes, and left to stand in a refrigerator (2 to 8°C) for 15 days.

### (3) Evaluation Method

One hundred µL samples were extracted from the liquid surface of the two glass test tubes, with one test tube being mixed by inversion prior to sampling while the other was kept still, each sample was then mixed with 900 µL (a 10-fold dilution) of 5 mM MOPS-NaOH (pH 7.0), and the absorbance at 600 nm and 800 nm was measured. The ratio of the absorbance at 600 nm relative to the absorbance at 800 nm (the 600/800 ratio) was calculated as an indicator representing the dispersibility of particles in the solution. The absorbance ratio of the absorbance values at 600 nm (prior to inversion mixing) / (after inversion mixing) was calculated as the latex particle content ratio (%) at the liquid surface.

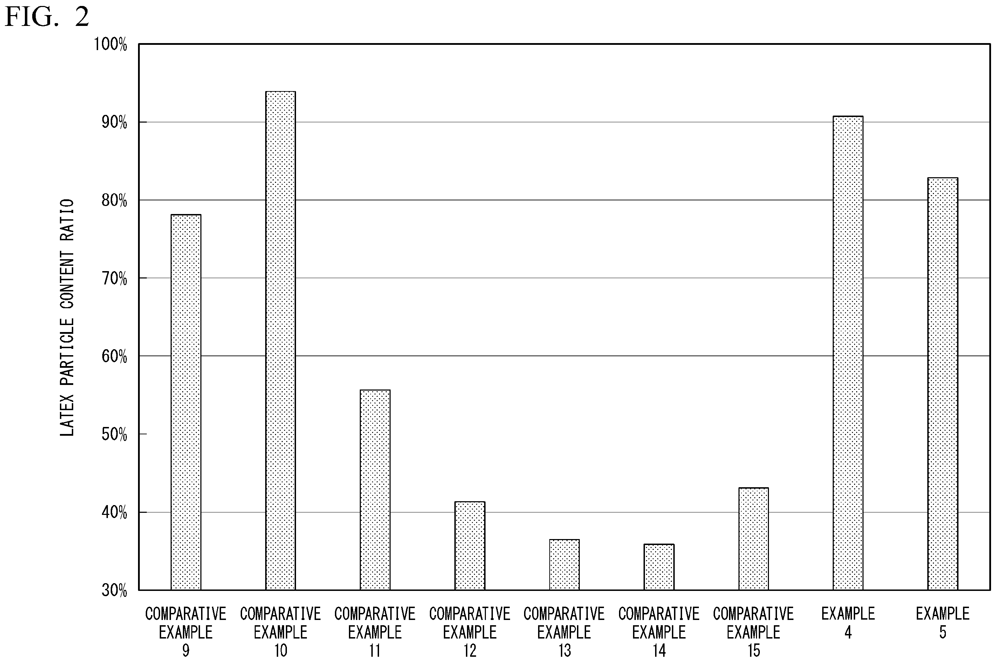

The measurement results are shown in Table 2. The latex particle content ratio prior to and after inversion mixing is shown in FIG. 2. The measured absorbance values at 600 nm and 800 nm were multiplied by 10, and the resulting absorbance of the raw solution was recorded in Table 2.

### <Comparative Examples 10 to 15, Examples 4 and 5>

With the exception of adding the component shown in the column labeled "Additive name" in Table 2 to the second reagent described in Comparative Example 9 in an amount sufficient to obtain the final concentration shown in the column labeled "Final concentration", measurements were conducted in the same manner as described above. The measurement results are shown in Table 2. The products listed below were used as additives.
- Sodium metaphosphate, pentasodium triphosphate, sodium diphosphate decahydrate: manufactured by FUJIFILM Wako Pure Chemical Corporation
- Sodium phosphate: manufactured by Tokyo Chemical Industry Co., Ltd.

**[Table 2]**

| | Additive | | Prior to inversion mixing | | | After inversion mixing | | | Latex particle content ratio |
|---|---|---|---|---|---|---|---|---|---|
| | Name of additive | Final concentration | Absorbance (600 nm) | Absorbance (800 nm) | 600/800 ratio | Absorbance (600 nm) | Absorbance (800 nm) | 600/800 ratio | |
| Comparative Example 9 | no additive | - | 4.146 | 1.860 | 2.23 | 5.308 | 2.412 | 2.20 | 78.1% |
| Comparative Example 10 | sucrose | 5% | 4.198 | 1.922 | 2.18 | 4.470 | 2.002 | 2.23 | 93.9% |
| Comparative Example 11 | sodium polyphosphate | 0.2% | 2.948 | 1.308 | 2.25 | 5.300 | 2.418 | 2.19 | 55.6% |
| Comparative Example 12 | sodium metaphosphate | 0.2% | 2.192 | 0.966 | 2.27 | 5.306 | 2.396 | 2.21 | 41.3% |
| Comparative Example 13 | pentasodium triphosphate | 0.2% | 1.924 | 0.848 | 2.27 | 5.278 | 2.418 | 2.18 | 36.5% |
| Comparative Example 14 | sodium phosphate | 0.2% | 1.854 | 0.864 | 2.15 | 5.170 | 2.342 | 2.21 | 35.9% |
| Comparative Example 15 | sodium diphosphate decahydrate | 0.2% | 2.212 | 1.002 | 2.21 | 5.132 | 2.364 | 2.17 | 43.1% |
| Example 4 | sodium hexametaphosphate | 0.01% | 4.800 | 2.222 | 2.16 | 5.292 | 2.406 | 2.20 | 90.7% |
| Example 5 | | 1.0% | 3.922 | 1.806 | 2.17 | 4.734 | 2.166 | 2.19 | 82.8% |

### (4) Results and Observations

In Test Example 2, the dispersibility of the latex particles was favorable under all of the conditions.

The latex particle content ratio was 78.1% in Comparative Example 9 containing no additive, but that value decreased in each of Comparative Examples 11 to 15, indicating states in which the particles were more prone to sedimentation. The only additives that exhibited a particle sedimentation suppression function were the sucrose of Comparative Example 10 and the sodium hexametaphosphate of Examples 4 and 5. The sucrose of Comparative Example 10 exhibited a sedimentation suppression function, but caused a deterioration in the measurement sensitivity (see Test Example 3 below), and was therefore not a preferred component. It was found that the sodium hexametaphosphate exhibited a sedimentation suppression function across a broad final concentration range from 0.01 to 1.0%. Phosphoric acid condensates are generally known as dispersants, but surprisingly, only the sodium hexametaphosphate exhibited a sedimentation suppression function for the particles having bound antibodies. In other words, the phosphoric acid condensates such as sodium polyphosphate, sodium metaphosphate, pentasodium triphosphate and sodium diphosphate decahydrate used in the comparative examples exhibited no sedimentation suppression function on the particles having bound antibodies, with only the sodium hexametaphosphate exhibiting a sedimentation suppression function for the particles having bound antibodies.

### [Test Example 3: Absorbance Measurement in LTIA: TARC measurement]

The second reagent of the LTIA measurement reagent and a first reagent described below were combined, and standard solutions containing known concentrations of added TARC antigen were measured as samples. By verifying the measurement sensitivity, the effect of the additive contained in the second reagent on the detection sensitivity was evaluated.

### <Comparative Example 16: No Additive>

The test method and evaluation method were as follows. Further, the reagent compositions are also described below.

### (1) Preparation of LTIA Measurement Reagent

An LTIA measurement reagent composed of the first reagent and second reagent described below was prepared.

### i) First Reagent

100 mM MOPS-NaOH (pH 7.5)
500 mM NaCl
0.5% BSA

### ii) Second Reagent

The second reagent used in Comparative Example 9 was used as is.

### (2) Samples

### TARC Standard Solutions

Sample 1: physiological saline solution (TARC 0 pg/mL)
Sample 2: TARC 661 pg/mL
Sample 3: TARC 1,856 pg/mL
Sample 4: TARC 4,746 pg/mL
Sample 5: TARC 9,760 pg/mL
Sample 6: TARC 20,421 pg/mL

### (3) Measurement Procedure

The first reagent and second reagent were combined, and a Hitachi automatic analyzer 3500 was used to measure the TARC concentration in the sample. Specifically, 120 µL of the first reagent was added to 2.4 µL of the sample, and after holding the resulting mixture at 37°C for 5 minutes, 40 µL of the second reagent was added and stirred. The change in absorbance accompanying aggregate formation was measured for the subsequent 5 minutes at a main wavelength of 570 nm and a secondary wavelength of 800 nm.

### <Comparative Example 17, Examples 6 and 7>

In Comparative Example 17, with the exception of using the second reagent used in Comparative Example 10 (containing a final sucrose concentration of 5.0%), measurements were conducted using the same method as that described above.

With the exception of using the second reagent used in Example 4 in Example 6, and using the second reagent used in Example 5 in Example 7 (containing final concentrations of sodium hexametaphosphate of 0.01% and 1.0% respectively), measurements were conducted using the same method as that described above.

### (4) Evaluation Method

For each of the absorbance values obtained by measuring the standard solutions, the value of the ratio of the absorbance value under each of the various conditions relative to the absorbance value for Comparative Example 16 (no additive) (namely, each absorbance value ÷ absorbance value for Comparative Example 16 (%)) was calculated and recorded in Table 3 as the "Measurement sensitivity (relative value)".

**[Table 3]**

| | Additive | | Measurement sensitivity (relative value) | | | | |
|---|---|---|---|---|---|---|---|
| | Additive name | Final concentration | Sample 2 | Sample 3 | Sample 4 | Sample 5 | Sample 6 |
| Comparative Example 16 | no additive | - | 100% | 100% | 100% | 100% | 100% |
| Comparative Example 17 | sucrose | 5% | 50% | 57% | 65% | 71% | 79% |
| Example 6 | sodium hexametaphosphate | 0.01% | 101% | 98% | 98% | 101% | 103% |
| Example 7 | | 1.0% | 92% | 83% | 84% | 85% | 90% |

### (5) Results and Observations

The sucrose that was added in Comparative Example 17 yielded a superior particle sedimentation suppression function in Test Examples 1 and 2, but as shown in Table 3, caused a deterioration in detection sensitivity. In particular, the sensitivity in the samples having a low TARC concentration (sample 2 and sample 3) was 50% and 57% respectively, approximately half of that observed in Comparative Example 16. In actual clinical investigations, where the detection of very small amounts of a target substance in a blood serum sample or the like that represents the analysis target is required, it can be stated that this type of reduction in measurement sensitivity for samples having a low concentration of the target substance is extremely undesirable. In contrast, the sodium hexametaphosphate of the present invention suppressed particle sedimentation without causing any deterioration in the measurement sensitivity, regardless of the TARC concentration, and can be said to be a component with extremely good applicability.

### Industrial Applicability

By using the latex particle dispersion of the present invention, sedimentation of the latex particles can be suppressed, meaning when the dispersion is used as the latex particle dispersion (second reagent) used in a latex turbidimetric immunoassay, measurements can be conducted without requiring re-dispersion of the latex particle dispersion (second reagent) by stirring or the like. Further, the on-board stability of the latex particle dispersion (second reagent) used in the latex turbidimetric immunoassay can be improved.

## Claims

1. A latex particle dispersion comprising latex particles and an aqueous medium,
further comprising at least one compound selected from the group consisting of hexametaphosphoric acid and salts thereof.

2. The latex particle dispersion according to Claim 1, which is used as a latex particle dispersion in a detection reagent used in detection of a target substance by latex turbidimetric immunoassay.

3. The latex particle dispersion according to Claim 1, which is used as a latex particle dispersion in a detection reagent containing a buffer solution and the latex particle dispersion that is used in detection of a target substance by latex turbidimetric immunoassay.

4. The latex particle dispersion according to Claim 1, wherein the salts are alkali metal salts.

5. The latex particle dispersion according to any one of Claims 1 to 4, wherein the aqueous medium comprises water.

6. The latex particle dispersion according to any one of Claims 1 to 4, wherein the latex particles are polymer particles derived from a monomer component comprising a monomer having a styrene structure or a vinylnaphthalene structure.

7. The latex particle dispersion according to any one of Claims 1 to 4, wherein the latex particles are latex particles onto which a substance capable of binding to a target substance has been supported.

8. The latex particle dispersion according to Claim 7, wherein the substance capable of binding to a target substance is an antigen or antibody for the target substance.

9. The latex particle dispersion according to any one of Claims 1 to 4, wherein an average particle size of the latex particles is within a range from 50 to 1,000 nm.

10. The latex particle dispersion according to any one of Claims 1 to 4, wherein the latex particles are included in an amount within a range from 0.001 to 0.20% by mass relative to a total mass of the latex particle dispersion.

11. A reagent kit used in detection of a target substance in a specimen by latex turbidimetric immunoassay, wherein
the reagent kit comprises the latex particle dispersion according to any one of Claims 1 to 4.

12. A method for detecting a target substance in a specimen by latex turbidimetric immunoassay, the method comprising:
a step of bringing the latex particle dispersion according to any one of Claims 1 to 4 and the specimen into contact.

13. A sedimentation suppressing agent for latex particles within a latex particle dispersion included in a detection reagent containing a buffer solution and the latex particle dispersion that is used in detection of a target substance by latex turbidimetric immunoassay, wherein
the sedimentation suppressing agent comprises at least one compound selected from the group consisting of hexametaphosphoric acid and salts thereof as an active ingredient.

14. The sedimentation suppressing agent according to Claim 13, wherein the salts are alkali metal salts.

15. A method for suppressing sedimentation of latex particles onto which a substance capable of binding to a target substance has been supported in a latex turbidimetric immunoassay, the method comprising:
a step of bringing the latex particles and at least one compound selected from the group consisting of hexametaphosphoric acid and salts thereof into contact within an aqueous medium.

16. The method for suppressing sedimentation according to Claim 15, wherein the salts are alkali metal salts.
